# EUROPEAN PATENT APPLICATION

(11) **EP 2 708 889 A1**
(43) Date of publication of application: **19.03.2014**
(21) Application number: 12184050.8
(22) Date of filing: 12.09.2012
(51) Int. Cl.: G01N 33/483, A61B 5/053, G01N 1/04

(54) **Method and device for obtaining a tissue sample and for characterizing the tissue sample by determining at least one electrical property**

(71) Applicant: Universität Leipzig, 04109 Leipzig (DE)
(72) Inventor: Klemm, Holger, 14776 Brandenburg (DE); Kurz, Randy, 04155 Leipzig (DE); Pänke, Oliver, 04105 Leipzig (DE); Robitzki, Andrea, 68519 Viernheim (DE)
(74) Representative: Kalhammer, Georg

(57) **Abstract**

The present invention relates to a method and a device (10) for obtaining a tissue sample (12) from a tissue of an animal, human, plant or marine organism or from a tissue culture (14) and for characterizing the tissue sample (12) by determining at least one electrical property of the tissue sample (12). The method is characterized by the steps of puncturing the tissue or tissue culture (14) with a hollow probe (16) having an open end, extracting the tissue sample (12) from the tissue or tissue culture (14) within the end (18) of the hollow probe (16), and determining the at least one electrical property of the extracted tissue sample (12) while the tissue sample (12) remains within the end (18) of the hollow probe (16). The device (10) comprises at least one hollow probe (16) having at least one open end (18) for puncturing the tissue or tissue culture (14) and for extracting the tissue sample (12) from the tissue or tissue culture (14) within the at least one end (18), at least one electrode (20) disposed within the hollow probe (16), and means (20, 22, 24) for determining the at least one electrical property of the extracted tissue sample (12) within the end (18) of the hollow probe (16), the means (20, 22, 24) comprising the at least one electrode (20).

## Description

### TECHNICAL FIELD

The present invention relates to a method and a device for obtaining a tissue sample from a tissue of an animal, human, plant or marine organism or from a tissue culture and for characterizing the tissue sample by determining at least one electrical property of the tissue sample.

The method and the device of the present invention are preferably used in the medical and pharmaceutical field, and the field of biodiversity concerning animals, plants and marine organisms, in particular for *in vitro* testing or analyzing or characterizing three-dimensional tissue or cell samples derived from living tissue or from tissue cultures.

### BACKGROUND OF THE INVENTION

In the pharmaceutical field testing of drugs or other pharmaceutically active test compounds is increasingly performed by in vitro treatment of three-dimensional tissue or cell cultures with the compounds to be tested and by analyzing the response of the samples. Medical applications demand on fast and selective, specific detection of sample properties and behavior. In the field of biodiversity sample preparation, selection and characterization concerning animals, plants and marine organisms demands on appropriate, fast selection, isolation and monitoring of cell and tissue samples. The analysis can comprise the measurement of intra- and extracellular physiological parameters and their alterations due to the treatment. An electrical sample property that can be determined is electrical impedance. The electrical impedance can be measured at different times during the treatment, or the electrical impedance of the treated sample can be compared with the impedance of the untreated sample. The impedance measurement which is also known as electrical impedance spectroscopy is a non-destructive method for monitoring cell growth, integrity, subcellular alterations and morphological changes of the sample.

Presently the preparation of a living tissue sample and the succeeding measurement of the electrical impedance or any other electrical property of the tissue sample are two separate laboratory processes. In a first process step the tissue sample is prepared manually by sectioning tissue with a scalpel. In a separate second process step the tissue sample is analyzed using a suitable analyzer.

An automation of the sample preparation is not yet possible so that the quality of a tissue sample derived from the first process step depends very much on the skill of the laboratory technician who performs the sectioning or micro-fragmentation of the tissue. Preparing the sample manually further requires at least one additional process step to transfer the tissue sample to the analyzer.

Due to these reasons the preparation and testing of tissue samples is a time consuming process.

EP 2 103 933 B1 discloses a device and method for measuring impedance in living organotypic tissue and a method for analyzing the effect of test compounds on living organotypic tissue samples by measuring the impedance of the tissue samples which are placed in a recording chamber between two electrodes.

WO 2006/047299 discloses an organotypic slice assay that is used to study neurodegenerative diseases. Brain tissue taken from an animal is sectioned, e.g. by use of a microtome. The sectioned brain tissue is used to establish an organotypic brain slice culture. The brain slice culture can be treated with different drugs and can be examined. Among others the examination can include electrophysiological recordings of action potentials by means of whole-cell voltage- and current clamp technique.

US 6,337,994 B1 discloses an electrical impedance probe that includes a hollow two-part trocar needle which is designed to acquire impedance measurements at its tip. An electrically conductive barrel is electrically separated from a stylet of the trocar needle so that their distal ends define two electrodes. Electrical impedance of the tissue contacting the electrodes is determined in order to distinguish between different types of tissue that the needle tip passes while inserting the needle.

Furthermore it is well known to use a biopsy needle or cannula for sampling cells from living tissue by puncturing the tissue with the hollow needle and by extracting a tissue sample from the tissue within the hollow needle.

### SUMMARY OF THE INVENTION

Therefore it is an object of the present invention to provide a method and device as set out in the beginning which can accelerate the process for obtaining and analyzing tissue samples and make it more economical.

It is another object of the present invention to provide a method and device as set out in the beginning which can provide for an automation and acceleration of the sampling and analyzing process, especially when a large number of tissue samples have to be obtained and analyzed.

It is a further object of the present invention to provide a method and device as set out in the beginning for reproducibly obtaining the tissue sample without the requirement of skilled labor.

In order to achieve these objects the present invention provides the following methods and devices:

[1] A method for obtaining a tissue sample from a tissue of an animal, human, plant or marine organism or from a tissue culture and for characterizing the tissue sample by determining at least one electrical property of the tissue sample, comprising the steps:
- puncturing the tissue or tissue culture (14) with a hollow probe (16) having at least one open end (18),
- extracting the tissue sample (12) from the tissue or tissue culture (14) within the at least one end (18) of the hollow probe (16), and
- determining the at least one electrical property of the extracted tissue sample (12) while the tissue sample (16) remains within the at least one end (18) of the hollow probe (16).

[2] The method according to item [1], wherein the hollow probe is a hollow needle (16) or cannula having a sharpened end (18).

[3] The method according to item [1] or [2], wherein said determining at least one electrical property of the tissue sample (12) comprises determining electrical impedance of the tissue sample (12).

[4] The method according to any one of the preceding items, wherein said at least one electrical property of the tissue sample (12) is determined through at least one electrode (20) disposed within the hollow probe (16) containing the tissue sample (16).

[5] The method according to item [4], wherein said at least one electrical property of the tissue sample (12) is determined through at least one further electrode (22) disposed in front of the end (18) of the hollow probe (16) containing the tissue sample (12).

[6] The method according to any one of the preceding items, further comprising the step: immersing the tissue or tissue culture (14) in an electrically conducting fluid before puncturing the tissue or tissue culture (14) with a hollow probe (16) and/or the step: immersing the hollow probe (16) with the tissue sample (12) into an electrically conducting fluid (56) before determining the at least one electrical property of the tissue sample (12).

[7] A device for obtaining a tissue sample from a tissue of an animal, human, plant or marine organism or from a tissue culture and for characterizing the tissue sample by determining at least one electrical property of the tissue sample, characterized by
- at least one hollow probe (16) having at least one open end (18) for puncturing the tissue or tissue culture (14) and for extracting the tissue sample (12) from the tissue or tissue culture (14) within the at least one end (18),
- at least one electrode (20) disposed within the hollow probe (16), and
- means (20, 22, 24) for determining the at least one electrical property of the extracted tissue sample (12) within the at least one end (18) of the hollow probe (16), the means (20, 22, 24) comprising the at least one electrode (20).

[8] The device according to item [7], wherein said at least one electrode (20) is in abutting contact with the tissue sample (12) within the at least on end (18) of the hollow probe (16).

[9] The device according to item [7], wherein said at least one electrode (20) is spaced from the tissue sample (12) within the at least one end (18) of the hollow probe (16).

[10] The device according to any one of items [7] to [9], characterized by a further electrode (22), wherein the further electrode (22) is disposed in front of the at least one end (18) of the hollow probe (16) containing the tissue sample (12) when the at least one electrical property is determined.

[11] The device according to any one of items [7] to [10], wherein the means (20, 22, 24) for determining the at least one electrical property comprises impedance measurement means (24) or impedance spectroscopy means.

[12] The device according to any one of items [7] to [11], wherein the hollow probe is a hollow needle (16) or cannula.

[13] The device according to any one of items [7] to [12], wherein the at least one open end (18) of the hollow probe (16) is delimited by an end face (36) in the shape of a truncated cone or pyramid and has a sharp rim (38).

[14] The device according to any one of items [10] to [14], characterized by at least one well (54) containing an electrically conducting fluid (56) and the at least one further electrode (22).

[15] The device according to any one of items [7] to [14], characterized by an array of hollow probes (16) for concurrently obtaining a plurality of tissue samples (12) and for characterizing the tissue samples (12) by determining the at least one electrical property.

### DETAILED DESCRIPTION OF THE INVENTION

With the method and device according to the invention it is possible to obtain a tissue sample and determine at least one electrical property of the tissue sample without the need to transfer the tissue sample which will result in an acceleration of the process and therefore make the process more economical. The process can be further accelerated and made even more economical by implementing the device with a plurality of hollow probes and by concurrently puncturing the tissue or tissue culture with the plurality of probes which are preferably moved in parallel and have their open ends disposed in one plane. A device with such an array of probes will also enable an automation of the sampling and analyzing process and will dispense with the present requirement of skilled labour. The tissue samples resulting from sequentially puncturing the tissue or tissue culture with a single probe or from concurrently puncturing the tissue or tissue culture with a plurality of identical probes will have exactly the same shape and dimensions and therefore the analysis of these samples will be very accurate and will yield highly reproducible results. Furthermore the extraction of the tissue samples and the determination of the at least one electrical property can be performed with one single device or apparatus.

The tissue culture can be a slice culture or an explant culture derived from any mammal, vertebrate and invertebrate species of embryonic, neonatal, postnatal, and adult individuals, plants, marine organisms.

According to a preferred embodiment of the invention the hollow probe is a hollow needle or cannula having a sharpened end. Preferably the hollow needle or cannula has a circular cross-section, however hollow needles or cannulas with a square or polygonal cross-section might also be used. In order to facilitate the extraction of the tissue sample especially when sampling a thin layer of tissue culture the rim which surrounds the open end of the hollow probe preferably has the shape of a truncated cone or pyramid respectively with a sharp edge at the tip. The sharp edge at the end of the hollow probe extends along the periphery of the end and is disposed in a plane perpendicular to the direction of movement of the hollow probe and will evenly penetrate the tissue culture.

Preferably the at least one electrical property of the tissue sample is the electrical impedance which is also called bio-impedance in the context of measuring the electrical impedance of living tissue. Electrical impedance spectroscopy, i.e. measurement of electrical impedance of living cells can be used to characterize the cell growth, integrity, sub-cellular alterations and morphological changes of cells as well as the cell environment in single and multilayered cell cultures. The measured electrical impedance is dependent from various cellular parameters such as the capacitance and resistance of the cell membranes as well as intracellular membranes of organelles, the resistance of the extracellular medium and intrinsic cytoplasm and the extracellular matrix.

In order to measure the impedance of an extracted tissue sample an alternate voltage with a variable frequency is applied to the tissue sample positioned within the at least one end of the hollow probe. Depending on the frequency of the applied voltage the measured impedance may be indicative for alterations of cellular compartments and cell membranes. Such impedance measurements can be used to monitor cell behavior in vitro.

For the measurement of the impedance of a tissue sample the device is provided with two electrodes which are preferably in electrical contact with and separated by the tissue sample. Furthermore the device comprises impedance measurement or spectroscopy means which are also referred to as impedance analyzer in the context of this application. The impedance measurement or spectroscopy means comprises a source of alternating voltage with a variable frequency range, e.g. a sine wave generator or oscillator, and means for determining the voltage drop and/or phase shift or phase angle across the tissue sample at different frequencies of the applied voltage.

According to a further preferred embodiment of the invention one of the two electrodes used for impedance measurement or impedance spectroscopy is disposed within the hollow probe so that it will be disposed on one side of the tissue sample once the latter has been extracted by means of the hollow probe and is located in the open end of the probe. Preferably the other electrode is separate from the hollow probe and is arranged in such a way that it can be disposed in front of the end of the hollow probe which contains the extracted tissue sample during the impedance measurement or impedance spectroscopy. In the context of this application one of the electrodes, where the alternating voltage is applied to, will be referred to as working electrode. The other electrode, where the voltage drop and/or phase shift or phase angle across the tissue sample is measured, will be referred to as counter or measurement electrode. However both electrodes can be used for applying the alternating voltage and for measuring the voltage drop and/or phase shift.

In order to determine the at least one electrical property of the tissue sample, i.e. in order to conduct the impedance measurement or impedance spectroscopy, the hollow probe with the tissue sample and the working electrode as well as the counter or measurement electrode are preferably immersed in an electrically conductive fluid. If the electrodes are spaced from the tissue sample during the impedance measurement or impedance spectroscopy the electrically conductive fluid provides for an electric contact between the tissue sample and the two electrodes.

If it is desired that the electrodes are in abutting electrical contact with the electrodes it is preferable to provide the inner surface with an electrically conducting coating which acts as one of the electrodes. In this case the other electrode preferably has a pointed tip facing the end of a complementary hollow probe such that the tip of the electrode will penetrate into the tissue sample when the hollow tube approaches this electrode. Advantageously only a part of the electrode which will penetrate into the tissue samples is conductive whereas the rest of the electrode is isolated so that it will not be in electrical contact with the fluid into which the hollow probe is immersed.

In order to avoid an electrical short circuiting between the two electrodes by the electrically conductive fluid the hollow probe is preferably an electrical isolator or provided with an electrically isolated coating.

When the inventive device comprises an array of a plurality of hollow probes which are used for extracting and analyzing tissue samples each of the hollow probes will contain at least one electrode. The other electrode which faces the open end of the hollow probe can be disposed on the bottom of a well filled with an electrically conducting fluid into which the hollow probe, the array of hollow probes or part of the array is introduced for impedance measurement. The use of small wells instead of one large well might avoid crosstalk between neighboring tissue samples. After having extracted the tissue samples from the tissue or tissue culture and before performing the impedance measurement or impedance spectroscopy the ends of the hollow probes with the tissue samples are automatically introduced into the well or wells and immersed in the electrically conductive fluid.

The present invention is illustrated by reference to the drawing figures, encompassing schematic views of two preferred embodiments of the invention, wherein:
Fig. 1 is a schematic view of a first embodiment of a device according to the invention for obtaining a tissue sample and for determining the electrical impedance of the tissue sample;
Figures 2a to 2f are schematic views of a method according to the invention for obtaining a tissue sample and for determining the electrical impedance of the tissue sample by use of the device of Fig. 1;
Fig. 3 is a schematic view of a second embodiment of a device according to the invention during the determination of the electrical impedance.

The devices 10 depicted in the drawing figures are used for extracting a tissue sample 12 from a slice of tissue culture 14 and for determining the electrical impedance of the tissue sample 12. Each of the depicted devices 10 comprises a hollow needle 16 having a lower open end 18, a first electrode 20 disposed within the hollow needle 16, a second electrode 22 disposed in front of the end 18 of the hollow needle 16 and an impedance analyzer 24. The upper end of the hollow needle 16 is also open.

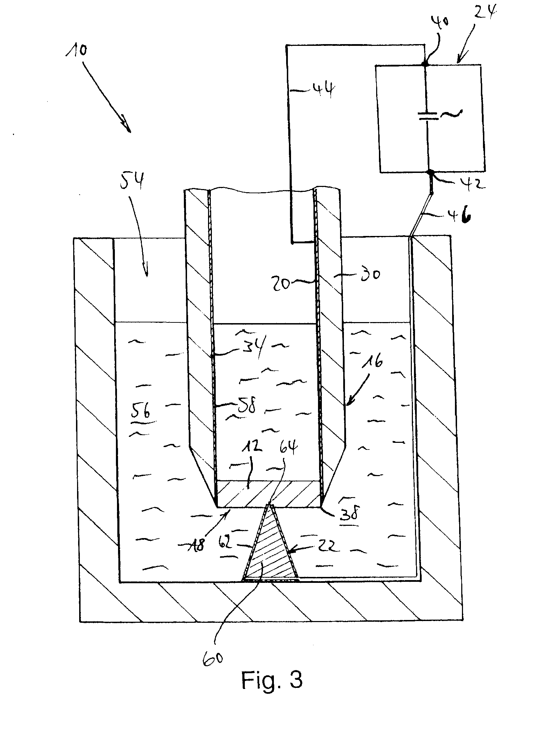

The slice of tissue culture 14 is a multilayered cell culture, e.g. of an organotypic human tissue, having a constant thickness from about 0.1 to 1.0 mm. The slice 14 is schematically depicted in Figs. 3 and 4 on a planar support 26. The slice 14 can be prepared by means of a tissue chopper or the like and laid down on the upper surface of the planar support 26. The planar support 26 can be made from a somewhat elastic or resilient material.

The hollow needles 16 of the devices 10 are provided with a tubular wall 30 having a cylindrical outer surface 32 and a cylindrical inner surface 34. The tubular wall 30 of the hollow needles 16 is made from a non-conductive material, e.g. quartz glass or stiff plastic material. The lower open end 18 of the hollow needles 16 is delimited on the outside by a cut end face 36 in the form of a steep truncated cone. The end face 36 connects the outer surface 32 with the inner surface 34 and tapers downwardly towards a sharpened annular rim 38 which is suitable for cutting tissue. The inner diameter of the hollow needles 16 is from about 0.2 to about 1 mm.

The impedance analyzer 24 of the devices 10 has a first and a second terminal 40, 42. The first terminal 40 is connected by a lead 44 to the first electrode 20 and the second terminal 42 is connected by al lead 46 to the second electrode 22. The impedance analyzer 24 comprises a sine wave generator (not shown) for applying an alternating voltage to the first terminal 40 and the first electrode 20, the frequency of the alternating voltage being variable from 100 Hz to 100 GHz. The impedance analyzer 24 further comprises means (not shown) for measuring the variance of the voltage drop and the phase shift or phase angle across a tissue sample 12 located in the open end 18 of a hollow needle 16 as a function of the frequency of the alternating voltage. In the following the first electrode 20 is referred to as working electrode and the second electrode 22 is referred to as counter or measurement electrode.

The working electrode 20 is a rod like cylindrical electrode which is disposed within the interior of the hollow needle 16 and extends along the axis of the hollow needle 16. The lower end 50 of the electrode 20 is spaced from the sharpened annular rim 38 where the distance is about 1 to 3 mms or more. The other end of the electrode 20 extends from the upper end of the hollow needle 16 and is connected by the lead 44 to the first terminal 40 of the impedance analyzer 24. The diameter of the electrode 20 is smaller than the diameter of the cylindrical inner surface 34 of the hollow needle 16 so that there is an annular space 52 between the inner surface 34 of the hollow needle 16 and the outer surface of the electrode 20.

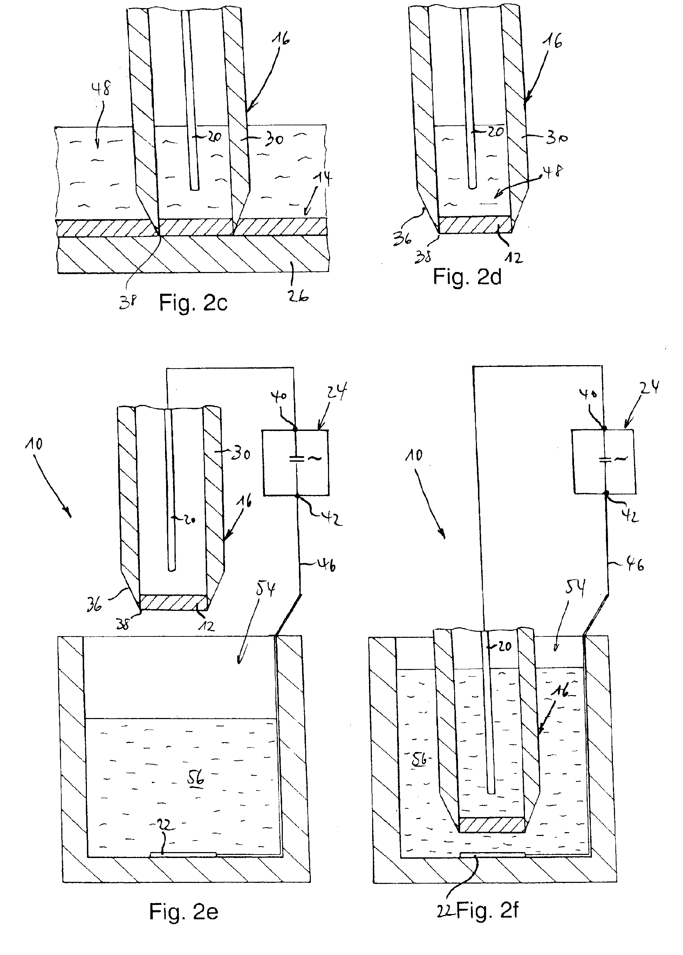

The counter electrode 22 is a flat circular electrode, however might have any suitable shape. As depicted in Figs. 2e and 2f the counter electrode 22 is disposed on the bottom of a small well 54 containing an electrically conductive fluid 56 that also maintains the viability of the tissue sample, e.g. a special culture medium or an isotonic saline solution. The diameter of the electrode 22 is about 0.5 mm and thus corresponds approximately to the inner diameter of the hollow needle 16, however might have any suitable dimension. The lead 46 from the counter electrode 22 to the second terminal 42 of the impedance analyzer 24 is isolated.

In the device of Fig. 3 the inner surface 34 of the tubular wall 30 of the hollow needle 16 is provided with a thin electrically conductive coating 58 which extends down to the sharpened annular rim 38 and acts as the working electrode 20. The coating 58 is preferably made from silver or gold or another metal having a good electrical conductivity. At the upper end of the hollow needle 16 the coating 38 is connected to the lead 44.

The counter electrode 22 in Fig. 3 is also disposed on the bottom of a small well 54 which contains an electrically conductive fluid 56, e.g. a special culture medium or an isotonic saline solution. The electrode 22 has a conical shape and comprises an inner core 60 made from an electrically conductive material, e.g. copper, and an outer coating 62 made from an electrically isolating material, e.g. lacquer or varnish. The outer coating 62 covers the core 60 with the exception of the tip 64 of the cone which faces the end 18 of the hollow needle 16 and the tissue sample 12 disposed within the open end 18. The isolated lead 46 connects the inner core 60 with the second terminal 42 of the impedance analyzer 24.

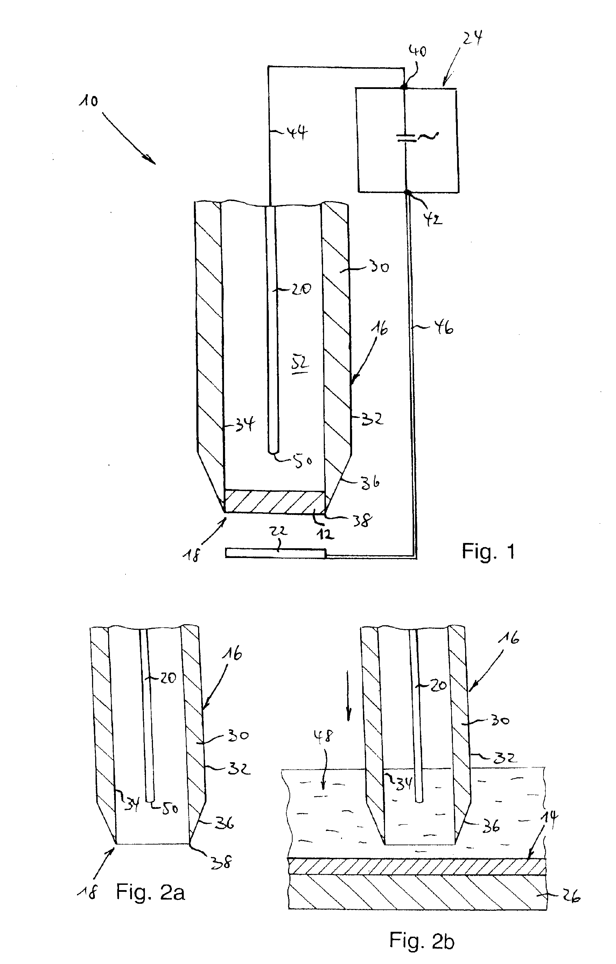

The extraction of a tissue sample 12 from the slice of tissue culture 14 and the determination of the electrical impedance of the tissue sample 12 will now be explained with regard to the device of Fig. 1, referring to Figs. 2a to 2f.

Fig. 2a depicts the hollow needle 16 with the working electrode 20 before starting the extraction of the tissue sample 12. For extracting the tissue sample 12 in a first step the hollow needle 16 is moved into a position where its open end 18 is located in a distance above the slice of tissue culture 14 which is immersed in an electrically conductive fluid 48, e.g. a special culture medium or an isotonic saline solution that maintains the viability of the tissue sample. The fluid 48 can be different from the fluid 56 or can be the same. In this position the open end 18 faces the slice of tissue culture 14 and the axis of the hollow needle 16 is perpendicular to the planar support 26 and the slice of tissue culture 14.

In a second step the hollow needle 16 is moved downwardly towards the slice of tissue culture 14 in the direction of its axis, as shown by the arrow in Fig. 2b. As soon as the open end 18 of the hollow needle 16 passes the surface of the electrically conductive fluid 48 the fluid 48 will enter into the annular space 52 between the inner surface 34 of the hollow needle 16 and the outer surface of the electrode 20 due to capillary forces and due to the fact that the opposite end of the hollow needle 16 is open.

When thereafter the sharpened annular rim 38 penetrates the slice of tissue culture 14 and gets into contact with the support 26 a tissue sample 12 in the form of small tablet is punched or cut from the slice of tissue culture 14 and is received within the open end 18 of the hollow needle 16 as depicted in Fig. 2c. The tissue sample 12 has a circular outline with a diameter corresponding to the inner diameter of the hollow needle 16 and a thickness corresponding to the thickness of the slice 14.

When the hollow needle 16 is moved away from the slice of tissue culture 14 in a third step the tissue sample 12 and the electrically conductive fluid 48 above the tissue sample 12 will remain within the end 18 of the hollow needle 16 as depicted in Fig. 2d. Due to the electrically conductive fluid 48 the working electrode 20 will be in electrical contact with the tissue sample 12.

In a fourth step the hollow needle 16 with the tissue sample 12 and the electrically conductive fluid is moved into a position where the end 18 of the hollow needle 16 with the tissue sample 12 and the fluid 48 is located above the well 54 which is partly filled with the electrically conductive fluid 56 and which is provided at the bottom with the counter electrode 22. This position is depicted in Fig. 2e.

In a fifth step the hollow needle 16 with the tissue sample 14 is moved downwardly into the well in the direction of its axis until the tissue sample 12 is spaced at a short distance from the upper surface of the counter electrode 22 as depicted in Fig. 2f. Due to the electrically conductive fluid 56 in the well 54 the counter electrode 22 will be also in electrical contact with the tissue sample 12, however separated from the working electrode 20 by the tissue sample 12. Therefore in the position of Fig. 2f any electrical current resulting from the alternating voltage applied to the terminal 40 will have to flow from the working electrode 20 through the tissue sample 12 to the counter or measurement electrode 22.

In this position the frequency of the alternating voltage applied by of the sine wave generator is varied, preferably from 1 Hz to 100 GHz, and the voltage drop and phase shift or phase angle at various frequencies are measured and recorded.

With the device 10 of Fig. 1 the voltage drop and the phase shift or phase angle across the tissue sample 12 are measured between two opposite faces of the flat circular tablet-shaped tissue sample 12 which faces are in electrical contact with the working electrode 20 and the counter or measurement electrode 22 respectively by means of the electrically conductive fluid 56.

In the device of Fig. 3 the working electrode 20 is in abutting electrical contact with the periphery of the flat circular tablet-shaped tissue sample 12 whereas the pointed tip 60 of the conical counter or measurement electrode 22 penetrates into the tablet-shaped tissue sample 12 at the centre of its bottom face. Therefore in this set-up the voltage drop and the phase shift or phase angle across the tissue sample 12 are measured between the rim and the centre of the sample 12.

The hollow needles 16 as depicted in Figs. 1 and 3 can be part of a plurality of identical hollow needles 16, for example an array (not shown) of hollow needles, which are disposed in parallel with a small distance between adjacent needles 16. The array can be moved between a working station and a measuring station (not shown). In the working station all of the hollow needles 16 of the array concurrently puncture a slice of tissue culture 14 immersed in an electrically conductive fluid 48 for extracting a plurality of tissue samples 12. In the measuring station the ends 18 of all of the hollow needles 16 are immersed in an electrically conductive fluid 56 within one single well or an array of wells. The number of wells can be any one from one to a number corresponding to the number of hollow needles 16 in the array. The or each well 54 contains an electrically conductive fluid 56 and at least one counter electrode 22.

In this case the impedance analyzer 24 comprises a multiplexing unit (not shown) which will successively apply the alternating voltage from the sine wave generator to each pair of working and counter electrodes 20, 22 so that in the impedance analyzer 24 each pair of electrodes 20, 22 pertaining to the same hollow needle 16 and associated well 54 can be individually addressed or multiplexed for measuring or recording the impedance of each tissue sample 12. The measurement or recording of the tissue samples 12 is conducted sequentially at a very high rate. The time needed for the impedance measurement or recording of each tissue sample 12 can be reduced further by applying a multi sinus alternating voltage that allows parallel impedance measurement at different frequencies which can be used for analysis of different cellular parameters.

## Claims

1. A method for obtaining a tissue sample from a tissue of an animal, human, plant or marine organism or from a tissue culture and for characterizing the tissue sample by determining at least one electrical property of the tissue sample, comprising the steps:
- puncturing the tissue or tissue culture (14) with a hollow probe (16) having at least one open end (18),
- extracting the tissue sample (12) from the tissue or tissue culture (14) within the at least one end (18) of the hollow probe (16), and
- determining the at least one electrical property of the extracted tissue sample (12) while the tissue sample (16) remains within the at least one end (18) of the hollow probe (16).

2. The method according to claim 1, wherein the hollow probe is a hollow needle (16) or cannula having a sharpened end (18).

3. The method according to claim 1 or 2, wherein said determining at least one electrical property of the tissue sample (12) comprises determining electrical impedance of the tissue sample (12).

4. The method according to any one of the preceding claims, wherein said at least one electrical property of the tissue sample (12) is determined through at least one electrode (20) disposed within the hollow probe (16) containing the tissue sample (16).

5. The method according to claim 4, wherein said at least one electrical property of the tissue sample (12) is determined through at least one further electrode (22) disposed in front of the at least one end (18) of the hollow probe (16) containing the tissue sample (12).

6. The method according to any one of the preceding claims, further comprising the step: immersing the tissue or tissue culture (14) in an electrically conducting fluid (56) before puncturing the tissue or tissue culture (14) with a hollow probe (16) and/or the step: immersing the hollow probe (16) with the tissue sample (12) in an electrically conducting fluid (56) before determining the at least one electrical property of the tissue sample (12).

7. A device for obtaining a tissue sample from a tissue of an animal, human, plant or marine organism or from a tissue culture and for characterizing the tissue sample by determining at least one electrical property of the tissue sample, **characterized by**
- at least one hollow probe (16) having at least one open end (18) for puncturing the tissue or tissue culture (14) and for extracting the tissue sample (12) from the tissue or tissue culture (14) within the at least one end (18),
- at least one electrode (20) disposed within the hollow probe (16), and
- means (20, 22, 24) for determining the at least one electrical property of the extracted tissue sample (12) within the at least one end (18) of the hollow probe (16), the means (20, 22, 24) comprising the at least one electrode (20).

8. The device according to claim 7, wherein said at least one electrode (20) is in abutting contact with the tissue sample (12) within the at least one end (18) of the hollow probe (16).

9. The device according to claim 7, wherein said at least one electrode (20) is spaced from the tissue sample (12) within the at least one end (18) of the hollow probe (16).

10. The device according to any one of claims 7 to 9, **characterized by** a further electrode (22), wherein the further electrode (22) is disposed in front of the at least one end (18) of the hollow probe (16) containing the tissue sample (12) when the at least one electrical property is determined.

11. The device according to any one of claims 7 to 10, wherein the means (20, 22, 24) for determining the at least one electrical property comprises impedance measurement means (24) or impedance spectroscopy means.

12. The device according to any one of claims 7 to 11, wherein the hollow probe is a hollow needle (16) or cannula.

13. The device according to any one of claims 7 to 12, wherein the at least one open end (18) of the hollow probe (16) is delimited by an end face (36) in the shape of a truncated cone or pyramid and has a sharp rim (38) .

14. The device according to any one of claims 10 to 13, **characterized by** at least one well (54) containing an electrically conducting fluid (56) and the at least one further electrode (22).

15. The device according to any one of claims 7 to 14, **characterized by** an array of hollow probes (16) for concurrently obtaining a plurality of tissue samples (12) and for characterizing the tissue samples (12) by determining the at least one electrical property.
